(19) Europäisches Patentamt
European Patent Office
Office européen des brevets

(11) Veröffentlichungsnummer: **0 026 311**
**B2**

(12) # NEUE EUROPÄISCHE PATENTSCHRIFT

(45) Veröffentlichungstag der neuen Patentschrift: **09.04.86**

(51) Int. Cl.⁴: **C 07 D 313/04**

(21) Anmeldenummer: **80104881.0**

(22) Anmeldetag: **16.08.80**

(54) **Verfahren zur Herstellung von sehr reinem epsilon-Caprolacton.**

(30) Priorität: **28.08.79 DE 2934659**

(43) Veröffentlichungstag der Anmeldung:
**08.04.81 Patentblatt 81/14**

(45) Bekanntmachung des Hinweises auf die Patenterteilung:
**30.03.83 Patentblatt 83/13**

(45) Bekanntmachung des Hinweises auf die Entscheidung über den Einspruch:
**09.04.86 Patentblatt 86/15**

(84) Benannte Vertragsstaaten:
**BE DE FR GB NL SE**

(56) Entgegenhaltungen:
**EP-A-0 020 952**
**DE-A-1 568 141**
**DE-B-1 086 686**
**DE-C-1 258 858**
**GB-A-841 839**
**GB-A-1 203 752**
**GB-A-1 364 526**
**JP-A-76 043 778**
**JP-A-78 034 789**

**Elements of Chemical Engineering (Mc Graw Hill 1936), S. 366**
**Techniques de l'Ingénieur (1965), S. J2620-1 bis J2620-8**
**Druckschrift "E-Caprolacton" (Degussa)**

(73) Patentinhaber: **Degussa Aktiengesellschaft, Weissfrauenstrasse 9, D-6000 Frankfurt am Main 1 (DE)**
Patentinhaber: **BAYER AG, Konzernverwaltung RP Patentabteilung, D-5090 Leverkusen 1 Bayerwerk (DE)**

(72) Erfinder: **Klenk, Herbert, Dr., Gruenaustrasse 19, D-6450 Hanau 9 (DE)**
Erfinder: **Wirthwein, Rolf, Dr., Fürstenbergstrasse 4, D-6450 Hanau 9 (DE)**
Erfinder: **Siekmann, Gerd, Wolfskaul 8, D-5000 Koeln 80 (DE)**
Erfinder: **Schwerdtel, Wulf, Dr., Hegelstrasse 9, D-5090 Leverkusen 1 (DE)**

EP 0 026 311 B2

LIBER, STOCKHOLM 1986

**Beschreibung**

Die vorliegende Erfindung betrifft ein kontinuierliches Verfahren zur technischen Gewinnung von farbstabilem ε-Caprolacton aus rohem ε-Caprolacton. Dabei soll nur rohes ε-Caprolacton, das durch die Umsetzung von Cyclohexanon mit organischen Percarbonsäuren gewonnen wurde, gereinigt werden.

Die organischen Percarbonsäure können in wässriger wie in organischer Lösung, bevorzugt aber in organischer Lösung, vorliegen.

Insbesondere betrifft die vorliegende Erfindung ein Verfahren zur Reinigung von rohem ε-Caprolacton, das durch die Umsetzung von organischen Lösungen der Perpropionsäure mit Cyclohexanon gewonnen wurde.

ε-Caprolacton ist ein wichtiges Ausgangsprodukt z.B. für die Herstellung von Polyesterolen, die anschliessend zu Polyuretanen umgesetzt werden. Eine evtl. Verfärbung des Lactons stört jedoch die Weiterverarbeitung, denn die Polymerisationsprodukte sind ebenfalls verfärbt.

Wird ε-Caprolacton nach vorbekannten Verfahren aus Cyclohexanon durch eine sogenannte Bayer-Villiger Oxidation, z.B. mit Percarbonsäuren, dargestellt (Meth. Chim. 5, (1975), S. 697 ff.), so fällt im allgemeinen ein Reaktionsgemisch an, das im wesentlichen ε-Caprolacton, die Basis-Säure und Cyclohexanon enthält.

Bei der Basis-Säure handelt es sich bevorzugt um eine niedermolekulare Carbonsäure. Sehr häufig liegt ein derartiges Reaktionsgemisch in Form einer Lösung vor, wobei als Lösungsmittel organische Lösungsmittel wie auch Wasser verwendet werden. Aus einer solchen Lösung oder einem solchen Gemisch wird ε-Caprolacton durch Destillation gewonnen. Die Aufarbeitungen können kontinuierlich oder diskontinuierlich geschehen.

So wird z.B. gemäß der Deutschen Patentschrift 1 258 858 aus einem Reaktionsgemisch, das Cyclohexanon, Essigsäure, Wasser und ε-Caprolacton enthält, nach dessen Destillation ε-Caprolacton in Ausbeuten um 80% erhalten. Die beste beschriebene Ausbeute liegt bei 90,5%.

Ein ganz ähnliches Verfahren wird in der DE—AS 1 693 027 beschrieben. Auch hier wird die Darstellung von Caprolacton nie mit einer höheren Ausbeute als 89%, bezogen auf Cyclohexanon, beschrieben.

Die DE—AS 1 086 686 betrifft ein Verfahren zur Darstellung von ε-Caprolacton in einem wasserfreien Medium. Nach der Destillation eines Gemisches, bestehend aus Aceton, Cyclohexanon, Essigsäure und ε-Caprolacton, betragen auch hier die Ausbeuten an ε-Caprolacton um 80%. Die beste beschriebene Ausbeute beträgt 90,3%.

In der DE—PS 1 216 283 und der Zusatzanmeldung DE—OS 1 643 146 wird als Oxidationsmittel Perameisensäure genannt, wobei eine Aufarbeitung entweder aus einer wässrigen oder einer organischen Lösung erfolgen kann. Die beste beschriebene Ausbeute an ε-Caprolacton liegt bei 84%.

Die Übersicht über diese bekannten Verfahren zeigt, dass ε-Caprolacton mit einer Ausbeute von mehr als 80% zwar dargestellt werden kann, es jedoch aber nur selten und schwer gelingt, eine Ausbeute von 90% zu erreichen. Dies ist natürlich ein erheblicher wirtschaftlicher Nachteil dieser Verfahren.

Auch in GB—A—841839, GB—A—1203752 und GB—A—1364526 sind Verfahren zur Herstellung von ε-Caprolacton aus Cyclohexanon durch Oxidation und anschließende destillative Aufarbeitung des Reaktionsgemischs beschrieben.

Aus JP—A—76/43778 und JP—A—78/34789 sind Verfahren zur weiteren Reinigung von so hergestelltem ε-Caprolacton durch Destillation bekannt.

Von einem solchen, nach den bekannten Verfahren hergestellten ε-Caprolacton wird erwartet, dass es eine gute Lagerbeständigkeit besitzt und insbesondere gegen Verfärbung oder gegen die Bildung von Säuren oder gegen Polymerisation bei Raum- oder höherer Temperatur stabil ist.

Dies ist aber nicht der Fall. So ist z.B. aus der DE—AS 1 493 317 bekannt, dass selbst hochgradig reine ε-Caprolactone bei der Lagerung nicht farbstabil sind. Ein frisch destilliertes ε-Caprolacton soll nur gering verfärbt sein, ändert aber seine Farbe bei der Lagerung.

In ähnlicher Weise liefert frisch destilliertes ε-Caprolacton bei der Herstellung von Polyesterdiolen Produkte mit geringer Verfärbung, während aus dem gleichen ε-Caprolacton nach zweiwöchiger Lagerung bei Raumtemperatur nur stark gefärbte Polyesterdiole erhalten werden.

Die Fachwelt war also sehr daran interessiert, ε-Caprolacton in einer Lagerstabilen Form zu erhalten. Sie konnte aber lange Zeit nichts anderes tun, als z.B. nach der DE—AS 1 493 317 selbst hochgradig gereinigte ε-Caprolactone vor ihrer Verwendung erneut zu destillieren. Über die Verluste an ε-Caprolacton bei dieser Destillation wurden keine Angaben gemacht, doch ist es ganz offensichtlich, dass dabei Verluste, wahrscheinlich sogar erhebliche Verluste, an ε-Caprolacton auftraten. Da die bisherigen Destillationsverfahren offensichtlich zu keinem befriedigenden Ergebnis geführt hatten, ging man dazu über, gewisse organische Substanzen, die ein stabilisierendes Moment für ε-Caprolacton besitzen, diesem zuzufügen.

So ist es z.B. nach der US—PS 3 227 730 bekannt, Triorganophosphite als Stabilisatoren für monomere ε-Caprolactone zu verwenden. Der Stabilisierungseffekt war aber nach Angaben der US—PS 3 274 216 nicht ausreichend, so dass nach dem Verfahren dieser Patentschrift ein Gemisch aus Triorganophosphiten und Alkylphenolen empfohlen wurde. Die Stabilisierungen wurden vorwiegend in einer Inertgasatmosphäre vorgenommen.

Nach der DE—AS 2 215 909 kann auch z.B. Triphenylphosphin als Stabilisator verwendet werden. Auch

wird die Stabilisierung in einer Inertgasatmosphäre vorgenommen.

Die Stabilisatoren werden bevorzugt in Mengen von 100—1 000 ppm verwendet, was in der Durchführung dieser Verfahren ein Nachteil sein kann, denn höhere Alkylphenole oder auch Organophosphorverbindungen sind meist keine gängigen Marktprodukte.

Bei der Verwendung von Stabilisatoren kann es ein Nachteil sein, dass dem ε-Caprolacton fremde Substanzen zugemischt werden, die gelegentlich drastisch das Verhalten oder die Reaktionseigenschaft des ε-Caprolactons beeinflussen können. Wie z.B. aus der Makrom. Chem. 56 (1962) 179—194 bekannt ist, wird ε-Caprolacton oft in Gegenwart von stark sauren oder stark basischen Substanzen polymerisiert. Nun ist es dem Fachmann geläufig, dass sowohl Phenole als auch Organophosphorverbindungen gegenüber Säuren oder Basen, gegebenenfalls noch in Gegenwart von Licht, Luft oder Schwermetallen, sehr reaktionsfreudig sein können und verschiedenartige, unter anderem auch stark farbige Zersetzungsprodukte ergeben. Auch beeinflusst z.B. nach Ansicht der DT—OS 2 160 405 eine solche Mischung aus Alkylphenolen und Triorganophosphiten bzw. ein Zusatz von Triorganophosphiten allein die physikalischen Eigenschaften von daraus hergestelltem Polyurethanprodukt in nachteiliger Weise. In Solchen Fällen kann also ein Inhibitor die Eigenschaften des ε-Caprolactons erheblich verschlechtern, und der Anwendungsbereich des ε-Caprolactons ist eingeschränkt.

Eine andere Methode zur Farbstabilisierung von ε-Caprolacton wird in der DE—PS 1 956 832 angegeben. Danach ist bekannt, dass man ein farbstabiles ε-Caprolacton erhält, wenn man das Lacton mit Sauerstoff und/oder einem oder mehreren Oxidationsmitteln und/oder einer oder mehreren im Vergleich mit dem Lacton schwerer flüchtigen Säuren behandelt und destilliert und gegebenenfalls das Lacton anschliessend noch einmal mit einer Menge sauerstoffabgebender Verbindung versetzt. Es besteht aber die Gefahr, dass das Caprolacton gerade unter diesen Bedingungen sehr leicht polymerisieren kann und dadurch Destillationsrückstände als Verluste erhalten werden.

Zusammenfassend muss also für die bisher bekannten Verfahren gesagt werden, dass die technische Darstellung von ε-Caprolacton nach obigen Verfahren durch zwei limitierende Momente eingeschränkt ist. Das ist einmal die ungenügende Produktreinheit, insbesondere die Farbinstabilität und ausserdem die ungenügende Wirtschaftlichkeit, bedingt durch ungenügende Ausbeuten. Werden zur Verbesserung der Farbstabilität Massnahmen durchgeführt, wie Destillation oder chemische Behandlungen — wie oben geschildert — gegenebenenfalls mit zusätzlichen Destillationen oder Zugabe von Stabilisatoren, so können diese Massnahmen nicht unbedingt erfolgreich sein und stellen zudem eine weitere Minderung der Wirtschaftlichkeit des betreffenden Verfahrens dar.

In der nach dem Prioritätsdatum veröffentlichten EP—A—20952 ist ein besonders vorteilhaftes Verfahren zur Herstellung von ε-Caprolacton aus Cyclohexanon durch Oxidation mit Perpropionsäure beschrieben, das darin besteht, daß man

a) eine Lösung von Perpropionsäure in einem organischen Lösungsmittel mit Cyclohexanon im Molverhältnis Cyclohexanon:Perpropionsäure wie 1,1—5:1 bei Temperaturen von 10—80°C umsetzt,

b) das erhaltene im wesentlichen aus ε-Caprolacton, Cyclohexanon, Propionsäure und organischem Lösungsmittel bestehende Reaktionsgemisch einer ersten Destillationseinheit zuführt, wo das organische Lösungsmittel für die Perpropionsäure als Destillat wiedergewonnen wird,

c) das Sumpfprodukt der ersten Destillationseinheit in eine zweite Destillationseinheit eingibt, wo die Propionsäure zusammen mit in Stufe a) nicht umgesetztem Cyclohexanon als Kopfprodukt gewonnen wird und ε-Caprolacton sowie gegebenenfalls hochsiedende Anteile getrennt voneinander unterhalb des Einlaufes in diese zweite Destillationseinheit abgezogen werden und

d) das aus Propionsäure und Cyclohexanon bestehende Destillat der zweiten Destillationseinheit in eine dritte Destillationseinheit überführt, wo ein aus Propionsäure und Cyclohexanon bestehendes Gemisch erhalten und als Destillat Propionsäure wiedergewonnen wird, worauf man,

e) das in Stufe d) anfallende Cyclohexanon und Propionsäure enthaltende Gemisch der Umsetzungsstufe a) wieder zuführt.

Es wurde nun gefunden, daß man aus dem nach diesem Verfahren erhaltenen ε-Caprolacton ein sehr reines, farbstabiles ε-Caprolacton gewinnen kann, wenn man das rohe ε-Caprolacton, das in Schritt c) unterhalb des Einlaufs in die zweite Destillationseinheit abgezogen wird, seinerseits einer vierten, bei 0,1 bis 500 mb betriebenen Destillationseinheit zuführt, an deren Kopf die Temperatur im Bereich von 10°C (bei 0,1 mb) bis 190°C (bei 500 mb) eingestellt wird, und als Kopfprodukt ein aus ε-Caprolacton und leichter siedenden Verunreinigungen bestehendes Gemisch abzieht, wonach man das Sumpfprodukt dieser Destillationseinheit einer fünften, bei 0,1 bis 500 mb betriebenen Destillationseinheit zuführt, an deren Kopf die Temperatur im Bereich von 15°C (bei 0,1 mb) bis 210°C (bei 500 mb) eingestellt wird und als Kopfprodukt das reine ε-Caprolacton abzieht.

Bevorzugt ist für das vorliegende Verfahren eine Perpropionsäurelösung in einem der oben genannten Lösungsmittel, vor allem in Benzol oder Dichlorpropan.

Als Destillationseinheit werden übliche Kolonnen mit Böden oder Füllkörpern eingesetzt.

Bei dem erfindungsgemässen Verfahren werden — wie gesagt — zwei zusätzliche Destillationseinheiten verwendet und zwar gestaltet sich das Verfahren z.B. so, dass das rohe ε-Caprolacton in die Mitte oder die obere Hälfte der vierten Fraktionierkolonne eingeführt wird.

Für eine solche Destillationskolonne üblicher Bauart sind z.B. Fraktionierkolonnen mit einer theoretischen Bodenzahl von mindestens 6 geeignet. Im allgemeinen können Kolonnen mit bis zu 200

theoretischen Böden verwendet werden, bevorzugt sind aber Destillationskolonnen mit 9—80 und ganz besonders bevorzugt solche mit 12—50 theoretischen Böden.

Zur Beheizung der Kolonnen können die üblichen Verdampfersysteme benutzt werden; besonders geeignet sind Fallfilmverdampfer.

Das Rücklaufverhältnis ist in ungewöhnlich weiten Grenzen variierbar, richtet sich aber nach den anderen Destillationsbedingungen. Üblicherweise werden Rücklaufverhältnisse von 0,5 bis 200 eingestellt, bevorzugt sind jedoch Rücklaufverhältnisse von 1 bis 25.

Obwohl die Kolonne so betrieben werden kann, dass ein grosser Teil des Einlaufs als Destillatentnahme abgezogen werden kann, lässt man bevorzugt nur geringe Mengen ab. Üblicherweise werden höchstens 10 Gew.-% des Einlaufs als Destillat entnommen, bevorzugt weniger als 1,5 Gew.-%. Da diese Destillatentnahme oft nicht mehr zur Gewinnung von ε-Caprolacton aufgearbeitet wird, werden hier also nur geringe Mengen an ε-Caprolacton verloren.

Die Gewinnung des Destillats und die Erzeugung des Rücklaufes kann auf die übliche Art und Weise erfolgen. Das heisst, es kann das Destillat z.B. total kondensiert werden und ein Teil davon als Rücklauf auf die Kolonne gegeben werden. Vorteilhafter ist jedoch die Verwendung eines Teilkondensationssystems wie z.B. eines Dephlegmatorsystems.

In einer solchen Destillatentnahme werden z.B. durch GC-MS-Untersuchung folgende Verbindungen festgestellt: ε-Caprolacton, Cyclohexanon, gegebenenfalls Propionsäure, daneben auch Hydroxicyclohexanon, Cyclohexanon und Cyclohexandion. Erstaunlicherweise fanden sich auch hochsiedende Stoffe wie dimeres Hydroxicyclohexanon sowie ein cyklisches Produkt folgender Struktur:

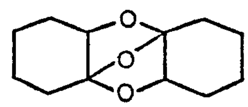

in der Destillatentnahme, d.h. in den Tiefsiedern, vor.

Eine mögliche Ausführungsform des Verfahrens beinhaltet nun die Rückführung dieser Destillatentnahme in das Verfahren zur Herstellung von ε-Caprolacton. Dadurch ist das ausgeschleuste ε-Caprolacton nicht verloren, genau so wie das ausgeschleuste Cyclohexanon. Die anderen Verbindungen gehen meist durch eigene Reaktionen während der ε-Caprolactonbildung in leicht abtrennbare Verbindungen über.

Der Teil das in die vierte Fraktionierkolonne eingeführten Stromes, der nicht als Destillatentnahme abgeführt wird, wird nun kontinuierlich aus dem Sumpf dieser Kolonne abgezogen und — wie gesagt — einer fünften Fraktionierkolonne in der Mitte oder der unteren Hälfte zugeführt. Auch hier ist eine Destillationskolonne üblicher Bauart geeignet, doch besitzt diese Kolonne bevorzugt weniger theoretische Böden als die vierte Kolonne. Im allgemeinen liegen hier 3—30 theoretische Trennstufen vor. Bevorzugt beträgt die theoretische Trennstufenzahl im Abtreiberteil 1—8 und im Verstärkerteil 2—22 theoretische Böden.

Bezüglich der Kondensation und Rücklauferzeugung bestehen keine Beschränkungen. Es kann also z.B. ein Teilkondensationssystem verwendet werden oder die aufsteigenden Dämpfe werden total kondensiert und ein Teil dieses Kondensates wird als Rücklauf auf die Kolonne gegeben.

Zur Beheizung auch dieser fünften Kolonne können die üblichen Verdampfersystem verwendet werden, besonders geeignet sind Fallfilmverdampfer oder Dünnschichtverdampfer.

Obgleich auch in dieser Kolonne der Druck weitgehend frei wählbar ist, ist es doch vorteilhaft, denselben oder einen ähnlichen Druck wie in der vierten Kolonne zu wählen.

Das Rücklaufverhältnis ist in weiten Grenzen variierbar und richtet sich nach den anderen Destillationsbedingungen. Üblicherweise werden jedoch geringere Rücklaufverhältnisse als in der vierten Kolonne verwendet. Im allgemeinen ist ein Rücklaufverhältnis von 0,1—5:1 ausreichend. Bevorzugt wählt man ein Rücklaufverhältnis, das im Bereich von 0,2—3:1 liegt.

Als Kopfprodukt dieser fünften Kolonne wird das sehr reine ε-Caprolacton gewonnen. Nach gaschromatografischer Analyse ist seine Reinheit im allgemeinen grösser als 99,99%.

Im Sumpf dieser fünften Kolonne können höher als ε-Caprolacton siedende Verbindungen festgestellt werden, doch sind dies meist weniger als 0,1 Gew.-% bezogen auf die der vierten Kolonne zugeführte Menge an ε-Caprolacton. Diese höhersiedenden Verbindungen werden kontinuierlich aus dem Sumpf der fünften Kolonne abgezogen, wobei sie aber üblicherweise als Lösung in ε-Caprolacton abgezogen werden. Bevorzugt liegen diese höher siedenden Verbindungen in Konzentrationen von 0,3 bis 30 Gew.-%, ganz besonders bevorzugt zwischen 0,6 und 15 Gew.-%, in dieser Lösung vor.

Die aus der fünften Kolonne ausgeschleusten Lösungen können verworfen werden, werden jedoch bevorzugt in das Verfahren zur Herstellung von rohem ε-Caprolacton zurückgeführt. Auf diese Weise kann das in der Lösung vorhandene ε-Caprolacton wieder gewonnen werden.

Ein wesentliches Kennzeichen des Verfahrens der EP—A—20952 stellt die Kreislaufführung eines aus Propionsäure und Cyclohexanon bestehenden Gemisches dar.

Vorzugsweise wird auch der Teilstrom, der nach dem erfindungsgemässen Verfahren aus dem Sumpf der fünften Kolonneneinheit abgezogen wird, direkt in diejenige Kolonne des Verfahrens der EP—A—20952 zurückgeführt, in der die Trennung von Cyclohexanon/Propionsäure von ε-Caprolacton und den Hochsiedern vorgenommen wird.

Der Ort für die Einführung dieses Stromes in die genannte Kolonne ist beliebig, doch wird er günstigerweise konzentrationsgerecht gewählt. Die Einführung des Stromes erfolgt daher vorzugsweise in der Nähe des Sumpfes der genannten Kolonne.

Der technische Fortschritt des erfindungsgemässen Verfahrens liegt einmal darin, dass durch die Erweiterung der zur Herstellung von ε-Caprolacton nach EP—A—20952 notwendigen Destillationsanlage um einige wenige Einheiten ein wirklich farbstabiles ε-Caprolacton erhalten werden kann.

Hinzu kommt, dass die Ausbeuten durch die mehrfachen Destillationen kaum vermindert werden, da die Verluste im allgemeinen nur etwa zwischen 1 und 2 Gew.-% liegen.

Es war nicht vorauszusehen, dass durch diese einfache Erweiterung und die dadurch mögliche Aufgliederung der Destillation des Rohproduktes in mehrere Einzeldestillationen, die Nebenprodukte, die die Lagerstabilität des ε-Caprolactons so sehr vermindern, praktisch aus dem Rohprodukt entfernt werden können und dass auf diese Weise ein wirklich farbstabiles ε-Caprolacton erhalten werden kann.

Die Erfindung wird ausserdem anhand folgender Beispiele erläutert:

An Figur 1 wird sowohl das erfindungsgemässe Verfahren allgemein dargestellt wie auch die speziellen Durchführungen der Beispiele 1 und 2.

### Beispiel 1: technisch

Die verwendete Apparatur besteht im kontinuierlichen Betrieb aus den zwei Destillationseinheiten 1 und 2, die das Zweikolonnen-Feinreinigungssystem darstellen.

In einem kontinuierlichen Versuch werden nun 20,48 kg/h rohes ε-Caprolacton über Leitung 3 der Destillationseinheit 1 zugeführt.

Die Destillationseinheit 1 besteht aus einer 8 m hohen Füllkörperkolonne mit Metall-Pallringen als Füllmaterial. Der Durchmesser der Kolonne beträgt 15 cm. Der Strom über 3 wird 2 m unterhalb des Kopfes der Kolonne eingeführt. Die Heizung dieser Kolonne erfolgt mit einem Fallfilmverdampfer (nicht gezeigt). Als Destillationskopf dient ein Dephlegmator (nicht gezeigt), der durch Kühlung so eingestellt wird, dass die aufsteigenden Kopfdämpfe so geteilt werden, dass ein Rücklaufverhältnis von 4:1 resultiert. Die Heizung wird dabei so eingestellt, dass pro Stunde ein Destillat über 4 von 0,25 kg resultiert.

Aus dem Sumpt dieser Destillationseinheit 1 wird währenddessen kontinuierlich die nicht als Destillat abgezogene Menge über 5 entnommen und der Mitte der Destillationseinheit 2 zugeführt. Die Destillationseinheit 2 besteht aus einer 3 m hohen Füllkörperkolonne mit einem Durchmesser von 20 cm und Metall-Pallringen als Füllmaterial. Diese Kolonne wird mit einem Dünnschichtverdampfer (nicht gezeigt) beheizt. Die aufsteigenden Kopfdämpfe werden in einem Kondensator total kondensiert und 12,8 kg/h Rücklauf über die Leitung 8 auf die Kolonne gegeben. Von dem Kondensat werden ausserdem 18,33 kg/h reines ε-Caprolacton über 6 entnommen. Aus dem Sumpf der Kolonne werden über den Dünnschichtverdampfer (nicht gezeigt) stündlich 1,9 kg über 7 abgezogen und in das Verfahren zur Herstellung von Caprolacton zurückgeführt, wo dieser Strom im Hochsieder und rohes ε-Caprolacton aufgetrennt werden kann. Eine genaue Analyse dieses Stromes zeigt, dass er zu 98,95 Gew.-% aus monomerem ε-Caprolacton und zu 1,05 Gew.-% aus höhersiedenden Verbindungen besteht.

Wie ersichtlich ist, gehen also durch die Destillationen nur 1,3 Gew.-% der zugeführten rohen Lactonmenge verloren.

Das ε-Caprolacton, das über 6 entnommen wurde, ist nach gaschromatografischer Analyse 99,99% rein (2 m 20% SE 52, He als Trägergas, 225° Ofentemperatur, WLD)

(2 m SE 52 = Säulenmaterial Silikongummi)

WLD = Wärmeleitfähigkeitsdetektor)

Es werden mit ihm die in Beispiel 3 und 4 beschriebenen Experimente durchgeführt.

### Beispiel 2

Es wird wie im Beispiel 1 verfahren, jedoch wird die Destillationseinheit 1 so verändert, dass die aufsteigenden Kopfdämpfe total kondensiert werden und in einen Destillatbehälter (nicht gezeigt) fliessen. Aus diesem Destillatbehälter wird pro Stunde 1 kg flüssige Phase auf den Kopf der Destillationseinheit 1 als Rücklauf gegeben und 0,25 kg/h als Destillatentnahme abgezogen.

Die übrigen Mengenflüsse und Verhältnisse sind dieselben wie in Beispiel 1.

Die gaschromatografische Analyse zeigt, dass in dem ε-Caprolacton über 6 ein Nebenprodukt mit einem Gehalt von 0,02% enthalten ist. Dieses Nebenprodukt wurde auch im Destillat über 4 gefunden, während es im rohen ε-Caprolacton über 3 nicht vorhanden ist.

Aus grösseren Mengen des Destillats über 4 wird nach mehrfachem Ausschütteln mit Wasser eine ölige Phase gewonnen, die destilliert wird. Bei 0,1 mbar und einer Siedetemperatur von 130°C geht ein Destillat über, aus dem beim Stehen in der Kälte wenige weisse Kristalle ausfallen. Diese werden aus Aceton umkristallisiert und besitzen einen Schmelzpunkt von 123°C. Ein Massenspektrum ergibt ein Molekulargewicht von 210.

Elementaranalyse
$C_{12}H_{18}O_3$
C ber. 68,54% gef. 68,62
H ber. 8,63% gef. 8,69

Im IR-Spektrum ist keine C = O Schwingung erkennbar. Das $^{13}$C-NMR Spektrum ergibt folgende Signale:
Werte in (ppm): 22,41 T; 22,89 T; 28,56 T; 32,58 T; 80,86 D; 107,85 S;
T = Triplett
D = Dublett
S = Singlett
Daraus ergibt sich folgende Struktur:

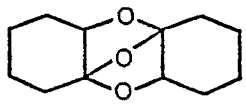

## Beispiel 3

Ein rohes ε-Caprolacton und ein ε-Caprolacton, das nach Beispiel 1 über 6 gewonnen wurde, besassen frisch nach ihrer Gewinnung eine Farbzahl von 10 unter Benutzung des ASTM-Standards D 1209—54.

Es wurde von ihnen je eine gewichtsmässig gleiche Probe unter Luft in eine gleiche Flasche abgefüllt und verschlossen. Die Luftmenge war in beiden Fällen die gleiche. Nach 4-wöchigem Stehen bei Raumtemperatur wurden die Proben erneut auf ihre Farbzahl untersucht. Die Probe ε-Caprolacton, die aus Leitung 6 entnommen wurde, besass immer noch eine Farbzahl von 10, die Probe rohes ε-Caprolacton dagegen eine Farbzahl von etwa 500.

## Beispiel 4

Polyester mit 2 Hydroxylgruppen (Polyesterdiole) wurden aus ε-Caprolacton durch Umsetzung mit einem Diol, wie Aethylenglykol, in Gegenwart eines Versterungskatalysators bei erhöhter Temperatur hergestellt. Die Farbwerte des erhaltenen Produktes waren folgende:

| Verwendetes ε-Caprolacton | (Farbzahl) Farbe des Polyesterdiols |
|---|---|
| 1. ε-Caprolacton aus über 6 frisch gewonnen | 10 |
| 2. rohes ε-Caprolacton frisch gewonnen | 10—20 |
| 3. ε-Caprolacton über 6 nach 4-wöchiger Lagerung | 10 |
| 4. rohes ε-Caprolacton nach 4-wöchiger Lagerung | etwa 200 |

## Patentansprüche

1. Verfahren zur Feinstreinigung von ε-Caprolacton das durch

a) Umsetzung einer Lösung von Perpropionsäure in einem organischen Lösungsmittel mit Cyclohexanon im Molverhältnis Cyclohexanon:Perpropionsäure wie 1,1—5:1 bei Temperaturen von 10—80°C,

b) Zuführung des erhaltenen, im wesentlichen aus ε-Caprolacton, Cyclohexanon, Propionsäure und organischem Lösungsmittel bestehenden Reaktionsgemisches zu einer ersten Destillationseinheit, in der das organische Lösungsmittel für die Perpropionsäure als Destillat wiedergewonnen wird,

c) Eingabe des Sumpfproduktes der ersten Destillationseinheit in eine zweite Destillationseinheit, in der die Propionsäure zusammen mit in Stufe a) nicht umgesetztem Cyclohexanon als Kopfprodukt gewonnen wird und ε-Caprolacton sowie gegebenenfalls hochsiedende Anteile getrennt voneinander unterhalb des Einlaufes in diese zweite Destillationseinheit abgezogen werden,

d) Überführung des aus Propionsäure und Cyclohexanon bestehenden Destillats der weiteren Destillationseinheit in eine dritte Destillationseinheit, in der ein aus Propionsäure und Cyclohexanon bestehendes Gemisch erhalten und als Destillat Propionsäure wiedergewonnen wird, und,

e) Rückführung des in Stufe d) anfallenden Cyclohexanon und Propionsäure enthaltenden Gemischs in die Umsetzungsstufe a)

erhalten worden ist, dadurch gekennzeichnet, daß man zur Gewinnung eines farbstabilen Produkts das rohe ε-Caprolacton, das in Schritt c) unterhalb des Einlaufs in die zweite Destillationseinheit abgezogen wird, seinerseits einer vierten, bei 0,1 bis 500 mb betriebenen Destillationseinheit zuführt, an deren Kopf die Temperatur im Bereich von 10°C (bei 0,1 mb) bis 190°C (bei 500 mb) eingestellt wird, und als Kopfprodukt ein aus ε-Caprolacton und leichter siedenden Verunreinigungen bestehendes Gemisch abzieht, wonach man das Sumpfprodukt dieser Destillationseinheit einer fünften, bei 0,1 bis 500 mb betriebenen Destillationseinheit zuführt, an deren Kopf die Temperatur im Bereich von 15°C (bei 0,1 mb) bis 210°C (bei 500 mb) eingestellt wird und als Kopfprodukt das reine ε-Caprolacton abzieht.

2. Verfahren nach Anspruch 1, dadurch gekennzeichnet, daß man höchstens 10 Gew.-% des Einsatzes in der vierten Destillationseinheit als Kopfprodukt abzieht.

3. Verfahren nach Anspruch 1, dadurch gekennzeichnet, daß man 50 bis 97 Gew.-% der Brüden der vierten Destillationseinheit kondensiert und als Rücklauf auf die vierte Destillationseinheit gibt und den nicht kondensierten Anteil in einem gesonderten Kondensator verflüssigt und als Destillat entnimmt.

4. Verfahren nach Anspruch 1 bis 3, dadurch gekennzeichnet, daß man das Destillat der vierten Destillationseinheit in die Herstellung von ε-Caprolacton wieder zurückführt.

5. Verfahren nach Anspruch 1 bis 4, dadurch gekennzeichnet, daß man das Sumpfprodukt der fünften Destillationseinheit in die Herstellung von ε-Caprolacton zurückführt.

**Revendications**

1. Procédé pour la purification fine de l'epsilon-caprolactone qui a été obtenue par:

a) réaction d'une solution d'acide perpropionique dans un solvant organique avec la cyclohexanone à un rapport molaire cyclohexanone/acide perpropionique de 1,1 à 5:1 à des températures de 10 à 80°C,

b) envoi du mélange de réaction obtenu, consistant essentiellement en epsilon-caprolactone, cyclohexanone, acide propionique et solvant organique, à une première unité de distillation, dans laquelle on récupère en distillat le solvant organique de l'acide perpropionique,

c) envoi du produit de pied de la première unité de distillation dans une deuxième unité de distillation dans laquelle on récupère en produit de tête l'acide propionique avec la cyclohexanone non convertie au stade a) et on évacue l'epsilon-caprolactone et le cas échéant les fractions à haut point d'ébullition, séparément, au-dessous de l'alimentation dans cette deuxième unité de distillation,

d) envoi du distillat de la deuxième unité de distillation, consistant en acide propionique et cyclohexanone, dans une troisième unité de distillation, dans laquelle on obtient un mélange consistant en acide propionique et cyclohexanone et on récupère en distillat l'acide propionique et

e) recyclage au stade de réaction a) du mélange contenant la cyclohexanone et l'acide propionique obtenu au stade d),

caractérisé en ce que, pour l'obtention d'un produit de coloration stable, on envoie à son tour l'epsilon-caprolactone brute qui est soutirée dans l'étape c) au-dessous de l'introduction dans la seconde unité de distillation, à une quatrième unité de distillation fonctionnant sous 0,1 à 500 mbar, en tête de laquelle la température est réglée dans l'intervalle de 10°C (sous 0,1 mbar) à 190°C (sous 500 mbar), et on évacue comme produit de tête un mélange consistant en epsilon-caprolactone et des impuretés volatiles, après quoi on envoie le produit de pied de cette unité de distillation à une cinquième unité de distillation fonctionnant sous 0,1 à 500 mbar, en tête de laquelle la température est réglée dans l'intervalle de 15°C (sous 0,1 mbar) à 210°C (sous 500 mbar) et on évacue comme produit de tête l'epsilon-caprolactone pure.

2. Procédé selon la revendication 1, caractérisé en ce que l'on évacue en produit de tête à la quatrième unité de distillation au maximum 10% du poids du produit mis en oeuvre.

3. Procédé selon la revendication 1, caractérisé en ce que l'on condense de 50 à 97% du poids des vapeurs de la quatrième unité de distillation et on les renvoie en reflux sur la quatrième unité de distillation, et on liquéfie la partie non condensée dans un condenseur séparé et on la prélève en tant que distillat.

4. Procédé selon les revendications 1 à 3, caractérisé en ce que l'on recycle à l'opération de préparation de l'epsilon-caprolactone le distillat de la quatrième unité de distillation.

5. Procédé selon les revendications 1 à 4, caractérisé en ce que l'on recycle le produit de pied de la cinquième unité de distillation à l'opération de préparation de l'epsilon-caprolactone.

**Claims**

1. Process for the super-fine purification of ε-caprolactone which has been obtained by

a) reacting a solution of perpropionic acid in an organic solvent with cyclohexanone in the molar ratio of cyclohexanone:perpropionic acid equal to 1.1—5:1 at temperatures of 10—80°C,

b) feeding the resulting reaction mixture, essentially consisting of ε-caprolactone, cyclohexanone, propionic acid and organic solvent, to a first distillation unit, in which the organic solvent for the perpropionic acid is recovered as distillate,

c) feeding the bottom product of the first distillation unit into a second distillation unit in which the propionic acid together with cyclohexanone not converted in stage a) is recovered as top product and ε-

caprolactone as well as any high-boiling constituents are taken off, separated from one another, below the inlet into this second distillation unit,

d) transferring the distillate, consisting of propionic acid and cyclohexanone, from the further distillation unit into a third distillation unit in which a mixture consisting of propionic acid and cyclohexanone is obtained and propionic acid is recovered as distillate and

e) recycling of the mixture, containing cyclohexanone and propionic acid, arising in stage d) into reaction stage a),

characterised in that to obtain a product which has a stable colour the crude ε-caprolactone which is taken off in step c) below the inlet into the second distillation unit is in turn referred to a fourth distillation unit, operated at 0.1 to 500 mb, at the top of which the temperature is set within the range from 10°C (at 0.1 mb) to 190°C (at 500 mb), and a mixture consisting of ε-caprolactone and more volatile impurities is taken off as the top product, after which the bottom product from this distillation unit is fed to a fifth distillation unit, operated at 0.1 to 500 mb, at the top of which the temperature is set in the range from 15°C (at 0.1 mb) to 210°C (at 500 mb) and the pure ε-caprolactone is taken off as the top product.

2. Process according to Claim 1, characterised in that at most 10% by weight of the feed is taken off as the top product in the fourth distillation unit.

3. Process according to Claim 1, characterised in that 50 to 97% by weight of the vapours of the fourth distillation unit are condensed and fed, as reflux, into the fourth distillation unit, while the uncondensed portion is liquefied in a separate condenser and taken off as distillate.

4. Process according to Claims 1 to 3, characterised in that the distillate of the fourth distillation unit is recycled to the preparation of the ε-caprolactone.

5. Process according to Claims 1 to 4, characterised in that the bottom product of the fifth distillation unit is recycled to the preparation of the ε-caprolactone.

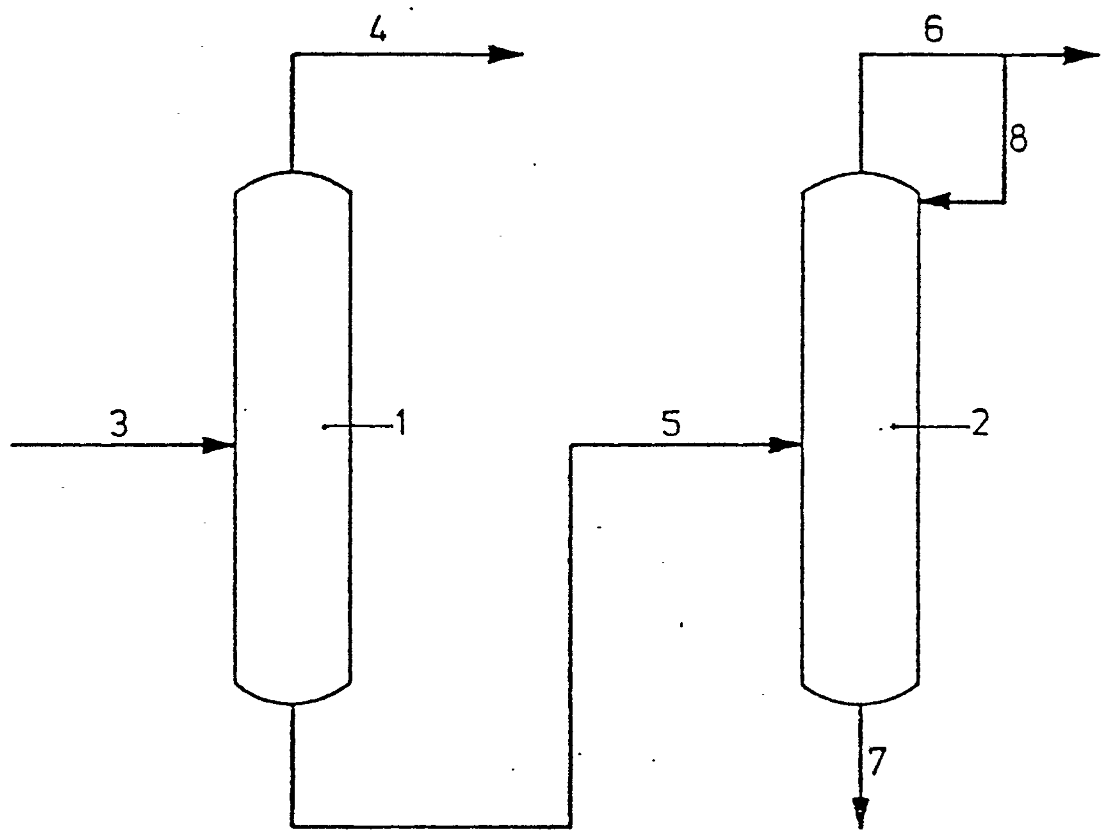


FIG.1